# FASCICULE DE BREVET EUROPEEN

(11) **EP 1 093 374 B1**
(45) Date de publication et mention de la délivrance du brevet: **16.01.2002**
(21) Numéro de dépôt: 99929401.0
(22) Date de dépôt: 05.07.1999
(51) Int. Cl.: A61K 33/14, A61K 35/08

(54) **SOLUTIONS SALINES HYPOOSMOTIQUES, LEUR PROCEDE DE PREPARATION ET MEDICAMENTS A BASE DE CES SOLUTIONS**
HYPOOSMOTISCHE SALZLÖSUNGEN, VERFAHREN ZU IHRER HERSTELLUNG UND PHARMAZEUTISCHER PRÄPARATE AUF BASIS DIESER LÖSUNGEN
HYPO-OSMOTIC SALINE SOLUTIONS, METHOD FOR PREPARING SAME AND MEDICINES BASED ON SAID SOLUTIONS

(30) Priorité: 03.07.1998 FR 9808568
(43) Date de publication de la demande: 25.04.2001
(73) Titulaire: LABORATOIRES GOEMAR S.A., 35400 Saint-Malo (FR)
(72) Inventeur: YVIN, Jean-Claude, F-35400 Saint Malo (FR); LEROY, Didier, F-35730 Pleurtuit (FR); TABARY, Olivier, F-51100 Reims (FR); JACQUOT, Jacky, F-51100 Reims (FR); PUCHELLE, Edith, F-51100 Reims (FR)
(74) Mandataire: Koch, Gustave
(86) Numéro de dépôt international: FR9901616
(87) Numéro de publication internationale: WO0001395

(56) Documents cités:
- FR-A- 2 735 980
- US-A- 5 466 680
- JONGEJAN RC ET AL: "Effects of changes in osmolarity on isolated human airways." J APPL PHYSIOL, APR 1990, 68 (4) P1568-75, XP002100212 UNITED STATES

## Description

L'invention a pour objet des solutions salines hypoosmotiques.

Elle vise également leur procédé de préparation et les médicaments à base de ces solutions.

On connaît déjà des solutions salines et leur mise en oeuvre en tant que médicaments.

On peut citer à cet égard le sérum physiologique et les solutions d'eau de mer isotonique; ces produits sont utilisés entre autres pour le lavage des plaies, des yeux et fosses nasales obstruées par des sécrétions muqueuses ou mucopurulentes.

La Société Demanderesse a mis au point de nouvelles solutions salines.

Ces nouvelles solutions salines sont caractérisées par le fait qu'elles sont hypoosmotiques et qu'elles présentent :
- un pH de 7,0 à 8,30,
- une résistivité de 52 à 370 Ω et de préférence de 57 à 85 Ω,
- une densité de 1,002 à 1,008,
- une teneur en matière sèche de 0,5% à 1%,
- une osmolarité comprise entre 100 mOs et 305 mOs/kg (milliosmoles/kg), de préférence entre 140 et 240 mOs/kg, et
- une constitution chimique dont les principaux éléments résultent du tableau I ci-après :

**TABLEAU I**

| | |
|---|---|
| Sodium (Na) | de 600 à 2000 mg/l |
| Potassium (K) | de 6 à 40 mg/l |
| Chlorures (Cl) | de 2000 à 5800 mg/l |
| Calcium (Ca) | de 200 à 300 mg/l |
| Magnésium (Mg) | de 1000 à 1200 mg/l |
| Sulfates (SO₄) | de 2000 à 3000 mg/l |

L'invention vise également l'utilisation en tant que médicament des solutions salines conformes à l'invention.

Elle a donc pour objet les médicaments ou compositions pharmaceutiques caractérisés par le fait qu'ils sont à base des solutions salines hypoosmotiques conformes à l'invention.

Plus particulièrement, l'invention a pour objet l'utilisation des solutions salines hypoosmotiques conformes à l'invention pour l'obtention d'un médicament destiné à un traitement propre à la prévention et à la limitation de la libération dans l'organisme des médiateurs chimiques responsables du déclenchement des phénomènes inflammatoires des muqueuses en général et plus particulièrement des muqueuses des voies respiratoires, notamment nasales et bronchiques, des muqueuses buccales, vaginales, intestinales et anales.

Il a été trouvé que les médiateurs chimiques en question sont libérés sous l'influence d'agents pro-inflammatoires qui, dans le cas notamment des voies respiratoires et buccales, font partie du groupe comprenant les substances d'origine bactérienne, les virus, bactéries, pollens et autres éléments irritants et allergisants transportés par l'air respiré.

L'invention a également pour objet l'utilisation des solutions salines hypoosmotiques conformes à l'invention pour l'obtention de médicaments destinés au traitement céruménolytique du conduit auditif externe, autrement dit à un traitement de dissolution des bouchons de cérumen.

L'invention a encore pour objet :
- un médicament à base d'une solution saline hypoosmotique selon l'invention, propre à prévenir et à limiter la libération, sous l'influence des substances d'origine bactérienne, des virus, bactéries, pollens et autres éléments irritants et allergisants transportés par l'air respiré, des médiateurs chimiques responsables du déclenchement des phénomènes inflammatoires des muqueuses en général et plus particulièrement des muqueuses des voies respiratoires, notamment nasales et bronchiques, des muqueuses buccales, vaginales, intestinales et anales, et
- un médicament céruménolytique à base d'une solution saline hypoosmotique selon l'invention.

Pour préparer les solutions hypoosmotiques, conformes à l'invention, on peut procéder comme indiqué ci-après.

On utilise comme matière première une eau de mer prélevée de préférence par une profondeur de 5 à 10 mètres dans une zone à forts mouvements de courant, et caractérisée par une teneur en sels supérieure à 32 g/l.

Cette eau est analysée, décantée puis rapidement :
- désodée par la technique d'électrodialyse jusqu'à ce que l'osmolarité soit amenée à une valeur comprise entre 100 et 305 mOs/kg,
- filtrée,
- stockée dans des conditions stériles, notamment en cuve en acier inoxydable.

Elle est de nouveau analysée à ce stade pour vérifier :
- sa stérilité,
- son osmolarité.

Enfin, elle peut être conditionnée de façon stérile dans un local spécialement traité en atmosphère contrôlée.

Les travaux qui ont permis d'illustrer certains aspects de l'invention comportent des expériences relatives à la capacité des solutions salines hypoosmotiques conformes à l'invention à prévenir et à limiter la libération des médiateurs chimiques responsables du déclenchement des phénomènes inflammatoires des muqueuses des voies respiratoires ; ces expériences montrent la supériorité des solutions salines hypoosmotiques conformes à l'invention par rapport à trois produits de référence bien connus, à savoir le sérum physiologique, le tampon PBS et les eaux de mer isotoniques.

Cette supériorité se traduit tant par une toxicité plus faible que par une activité améliorée du point de vue des effets recherchés.

Les toxicités respectives des solutions salines hypoosmotiques conformes à l'invention, du sérum physiologique, du tampon PBS et de l'eau de mer isotonique ont été définies par le biais d'une étude comparative portant sur la viabilité des cellules respiratoires humaines, c'est-à-dire des cellules que l'on rencontre dans les muqueuses respiratoires, en présence des solutions hypoosmotiques d'une part et des trois produits de référence d'autre part.

Pour faire cette étude, on a utilisé des cellules épithéliales glandulaires respiratoires humaines issues de prélèvements bronchiques.

On a ensemencé ces cellules épithéliales respiratoires à la même densité cellulaire dans des plaques de culture de 12 puits.

On les a cultivées pendant 48 heures dans un milieu de culture DMEM/F12 supplémenté d'une part avec 2% d'un nutriment constitué par le produit commercialisé sous la marque ULTROSER G et d'autre part avec une dose d'antibiotiques suffisante pour éviter la contamination bactérienne ; on obtient ainsi des cultures en monocouche homogènes et confluentes, soit 310 000 cellules par puits de culture après 48 heures de culture.

Au moment où la confluence est atteinte, les cellules sont lavées avec une solution physiologique (PBS/Ca²⁺ , pH 7,4) et mises en présence d'une part de huit solutions salines hypoosmotiques conformes à l'invention et d'autre part des trois produits de référence constitués par le sérum physiologique, le tampon PBS et l'eau de mer isotonique désignée par K ; on a également utilisé le susdit milieu de culture cellulaire de référence connu sous la désignation DMEM/F12.

Les huit solutions hypoosmotiques testées sont caractérisées respectivement par des résistivités égales à 57,30 Ω, 64,93 Ω, 74,34 Ω, 84,90 Ω, 107 Ω, 134,40 Ω, 192,30 Ω et 361 Ω et désignées respectivement par les lettres J, I, H, G F, E, D et C.

Les principaux éléments constitutifs des solutions hypoosmotiques G, H, I et J apparaissent dans le tableau II ci-après.

**TABLEAU II**

| Constituants | Unités | G | H | I | J |
|---|---|---|---|---|---|
| Sodium (Na) | mg/l | 600 | 765 | 1130 | 1370 |
| Potassium (K) | mg/l | 6,9 | 11,2 | 20,8 | 27,4 |
| Chlorures (Cl) | mg/l | 2556 | 2930 | 3797 | 4248 |
| Calcium (Ca) | mg/l | 265 | 290 | 310 | 330 |
| Magnésium (Mg) | mg/l | 1100 | 1130 | 1200 | 1200 |
| Sulfates (SO₄) | mg/l | 2570 | 2570 | 2570 | 2600 |

A l'issue de plusieurs périodes d'incubation, c'est-à-dire de contact cellulaire, à savoir 16 heures et 24 heures, on a évalué la viabilité cellulaire en présence des milieux dont il vient d'être question.

Cette viabilité cellulaire est exprimée par le pourcentage de cellules viables à la fin de chaque période d'incubation dans les solutions salines hypoosmotiques selon l'invention, dans le sérum physiologique, dans le tampon PBS et dans le milieu K, par rapport au nombre de cellules viables dans le milieu de culture DMEM/F12 servant de milieu de référence.

Elle a été évaluée à l'aide du test d'exclusion dit au bleu de Trypan, en solution à 0,4%, suivi d'un comptage cellulaire avec une cellule de Malassez.

Conformément au test en question, on détache les cellules du support de culture en utilisant de la trypsine ; les cellules vivantes sont ensuite comptées dans une solution aqueuse à 0,4% de bleu de Trypan ; en effet, le bleu de Trypan est un colorant cellulaire qui ne pénètre pas à l'intérieur d'une cellule vivante, seules les cellules non viables laissant entrer le bleu de Trypan.

Le nombre de cellules vivantes dans le milieu de culture DMEM/F12 étant par définition rapporté à 100, il est possible d'exprimer la viabilité cellulaire dans les autres milieux, en pourcentage par rapport audit milieu de culture.

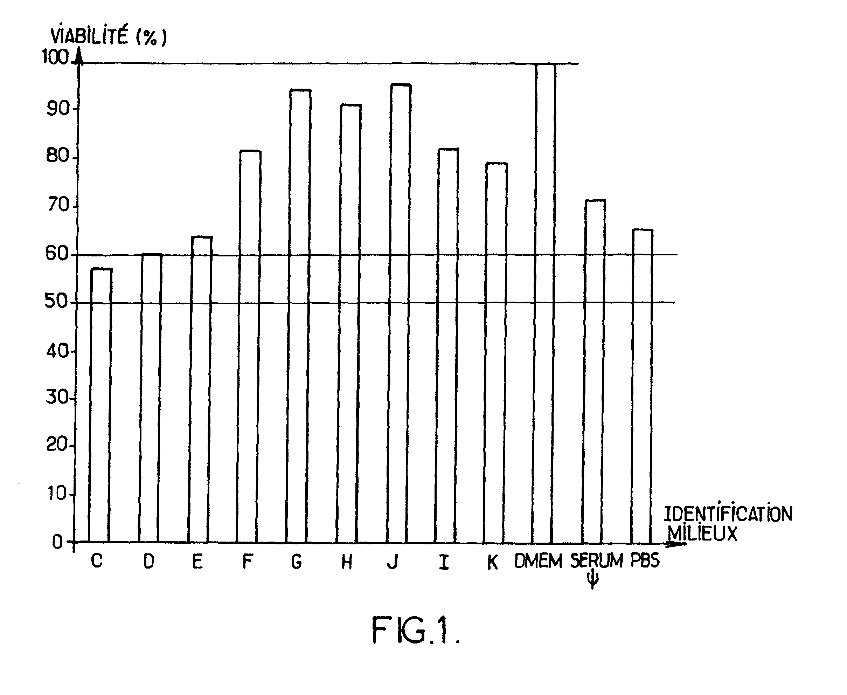

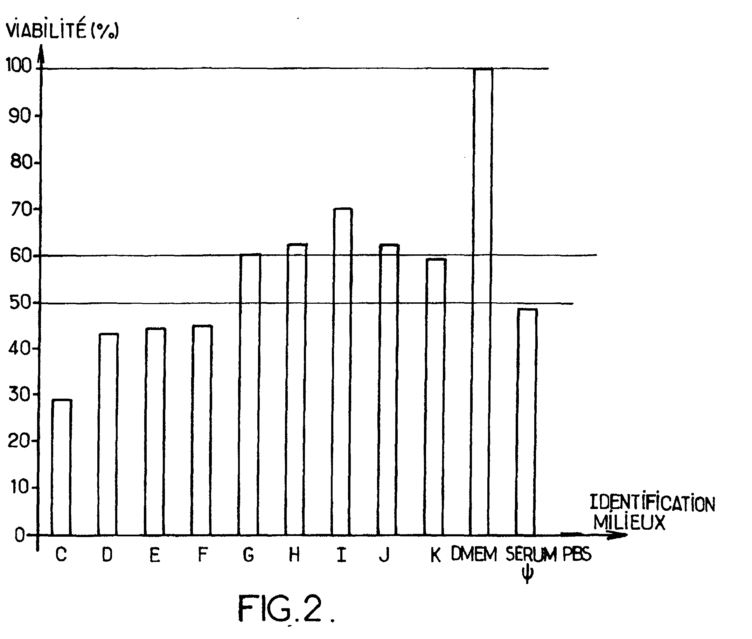

Les résultats enregistrés sont réunis dans les figures 1 et 2 qui montrent respectivement les résultats enregistrés après 16 heures et 24 heures de contact, à savoir le pourcentage de cellules vivantes après contact avec chacune des solutions hypoosmotiques conformes à l'invention et chacun des produits de référence, étant rappelé que la viabilité dans le milieu de culture DMEM/FI2 est de 100%.

Les pourcentages en question sont également réunis dans le tableau III :

**TABLEAU III**

| Pourcentage de cellules viables | | | | | | | | | | | | |
|---|---|---|---|---|---|---|---|---|---|---|---|---|
| | C | D | E | F | G | H | I | J | K | DME M | Sérum physiologique | PBS |
| 16 heures | 57 | 60 | 64 | 80 | 94 | 91 | 95 | 82 | 79 | 100 | 72 | 66 |
| 24 heures | 29 | 43 | 44 | 45 | 60 | 62 | 70 | 62 | 59 | 100 | 49 | 0 |

L'examen des figures 1 et 2 et des valeurs réunies dans le tableau III montre que les solutions salines hypoosmotiques préférentielles conformes à l'invention sont les solutions G, H, I et J qui, sur des périodes d'incubations de 16 et 24 heures, permettent une meilleure viabilité cellulaire que les produits de référence.

Pour la mise en évidence de la capacité des solutions salines hypoosmotiques conformes à l'invention, à prévenir et à limiter la libération, sous l'influence des agents pro-inflammatoires comprenant les substances d'origine bactérienne, les virus, les bactéries et tous autres éléments irritants et allergisants transportés par l'air respiré, des médiateurs chimiques responsables du déclenchement des phénomènes inflammatoires des muqueuses des voies respiratoires humaines, on soumet des cultures cellulaires provenant des muqueuses en question à un ensemble d'expériences comprenant une étape de mise en présence avec des agents pro-inflammatoires et une étape d'incubation des cultures cellulaires ainsi traitées dans un milieu de contrôle, dans des milieux de référence et dans les solutions hypoosmotiques selon l'invention.

Il a plus particulièrement été montré que, sous l'action de certains agents pro-inflammatoires d'origine bactérienne, tels que par exemple le lipopolysaccharide de P. aeruginosa ou LPS, sérotype 10, utilisé à la concentration de 1 µg/ml, les cellules épithéliales, glandulaires, respiratoires humaines libèrent des médiateurs chimiques tels que l'interleukine-8, qui déclenchent les phénomènes inflammatoires.

Et la Société Demanderesse a trouvé que, de façon surprenante et inattendue, une très forte prévention et limitation de la libération des médiateurs chimiques, en l'occurrence l'interleukine-8, est obtenue lorsqu'après une stimulation par des agents pro-inflammatoires, on incube les cultures cellulaires concernées dans les solutions salines hypoosmotiques utilisées selon l'invention.

Les susdites expériences ont été effectuées sur des cultures de cellules sécrétoires glandulaires isolées de la sous-muqueuse bronchique humaine.

Les cellules en question sont cultivées dans un milieu constitué, par exemple, par celui connu sous la désignation RPMI 1640 ; ce milieu est additionné de 2% de milieu ULTROSER G ; lorsque les cultures ont atteint une confluence de 90%, on introduit l'agent pro-inflammatoire identifié plus haut, puis on élimine le milieu de culture.

Après lavage des cellules ainsi traitées, on les réincube :
- dans le même milieu de culture RPMI 1640 seul qui joue le rôle de milieu de contrôle,
- dans le sérum physiologique (milieu de référence),
- dans l'eau de mer isotonique (milieu de référence),
- dans les solutions salines hypoosmotiques selon l'invention identifiées plus haut et désignées par H, I et J.

La production d'interleukine-8 par les cellules épithéliales glandulaires est estimée par la méthode de dosage ELISA (en utilisant le kit de dosage vendu sous la marque MEDGENIX DIAGNOSTIC , Belgique) après une période de temps de contact d'une heure dans les surnageants des milieux identifiés ci-dessus.

Les résultats enregistrés d'une part pour les trois solutions hypoosmotiques H, I et J conformes à l'invention et d'autre part pour le milieu de culture DMEM/F12, ainsi que pour les milieux de référence, à savoir le sérum physiologique et l'eau de mer isotonique, sont :
- de 282 pg/ml/heure dans le cas du milieu DMEM/F12,
- de 76 pg/ml/heure dans le cas du sérum physiologique,
- de 42 pg/ml/heure dans le cas d'eau de mer isotonique,
- de 29 pg/ml/heure dans le cas de la solution J,
- de 16 pg/ml/heure dans le cas de la solution I, et
- de 7 pg/ml/heure dans le cas de la solution H.

Ces résultats montrent la supériorité des solutions conformes à l'invention du point de vue de leur capacité à prévenir et à limiter la libération sous l'influence des agents pro-inflammatoires des médiateurs chimiques responsables du déclenchement des phénomènes inflammatoires des muqueuses.

Il a été possible de confirmer ces résultats par des expériences réalisées sur la culture d'une lignée cellulaire de l'épithelium respiratoire de surface (ligne respiratoire 16-HBE).

Dans la pratique, et dans le cadre de leur utilisation pour l'obtention des médicaments dont il vient d'être question, les solutions salines hypoosmotiques conformes à l'invention peuvent être administrées sous forme d'aérosols ou de nébulisats ou par instillation sous forme de gouttes.

Il s'agit de compositions pharmaceutiques ou médicaments destinés au traitement prévenant et limitant la libération des médiateurs chimiques responsables du déclenchement des phénomènes inflammatoires des muqueuses des voies respiratoires humaines survenant dans les affections rhinopharyngées et pulmonaires.

Les solutions salines hypoosmotiques conformes à l'invention peuvent être présentées sous la forme d'aérosols pour administration nasale et buccale à action locale exerçant son effet sur les muqueuses des voies respiratoires humaines, notamment au niveau des fosses nasales et des bronches.

La posologie habituelle peut être de 2 bouffées espacées d'une trentaine de secondes, répétées deux à trois fois par jour en inhalation buccale selon l'état et l'âge du patient.

Dans le cas d'une inhalation nasale, elle est habituellement d'une à deux bouffées dans chaque narine 3 à 4 fois par jour selon l'état et l'âge du patient.

On peut également envisager d'utiliser telles quelles les solutions hypoosmotiques conformes à l'invention en vue de prévenir et de limiter la libération des médiateurs chimiques responsables du déclenchement des phénomènes inflammatoires dans le cas des muqueuses buccales, vaginales, intestinales et anales.

La posologie sera adaptée en fonction du type d'affection traitée.

Dans le cadre de leur utilisation pour l'obtention de médicaments pour le traitement céruménolytique du conduit auditif externe, s'agissant alors de médicaments céruménolytiques, les solutions salines hypoosmotiques conformes à l'invention, peuvent être appliquées par pulvérisation ou par instillation sous forme de gouttes dans le conduit auditif externe de l'oreille.

En présence de bouchons de cérumen, on peut avantageusement les appliquer à raison de deux pulvérisations ou instillations quotidiennes pendant une période 3 à 4 jours.

A titre d'hygiène régulière, on peut les appliquer avantageusement deux à trois fois par semaine, et plus dans le cas des porteurs de prothèses auditives, afin de favoriser le bon fonctionnement de l'appareil.

## Revendications

1. Solutions salines **caractérisées par le fait qu'**elles sont hypoosmotiques et qu'elles présentent :
- un pH de 7,0 à 8,30,
- une résistivité de 52 à 370 Ω et de préférence de 57 à 85 Ω,
- une densité de 1,002 à 1,008,
- une teneur en matière sèche de 0,5% à 1%,
- une osmolarité comprise entre 100 mOs et 305 mOs/kg (milliosmoles/kg), de préférence entre 140 et 240 mOs/kg, et
- une constitution chimique dont les principaux éléments résultent du tableau I ci-après :
**TABLEAU I**
| | |
|---|---|
| Sodium (Na) | de 600 à 2000 mg/l |
| Potassium (K) | de 6 à 40 mg/l |
| Chlorures (Cl) | de 2000 à 5800 mg/l |
| Calcium (Ca) | de 200 à 300 mg/l |
| Magnésium (Mg) | de 1000 à 1200 mg/l |
| Sulfates (SO₄) | de 2000 à 3000 mg/l |

2. Médicament **caractérisé par le fait qu'**il est à base d'une solution saline hypoosmotique et qu'il présente :
- un pH de 7,0 à 8,30,
- une résistivité de 52 à 370 Ω et de préférence de 57 à 85 Ω,
- une densité de 1,002 à 1,008,
- une teneur en matière sèche de 0,5% à 1%,
- une osmolarité comprise entre 100 mOs et 305 mOs/kg (milliosmoles/kg), de préférence entre 140 et 240 mOs/kg, et
- une constitution chimique dont les principaux éléments résultent du tableau I ci-après :
**TABLEAU I**
| | |
|---|---|
| Sodium (Na) | de 600 à 2000 mg/l |
| Potassium (K) | de 6 à 40 mg/l |
| Chlorures (Cl) | de 2000 à 5800 mg/l |
| Calcium (Ca) | de 200 à 300 mg/l |
| Magnésium (Mg) | de 1000 à 1200 mg/l |
| Sulfates (SO₄) | de 2000 à 3000 mg/l |

3. Utilisation en tant que médicament des solutions salines selon la revendication 1.

4. Utilisation des solutions salines hypoosmotiques selon la revendication 1 pour l'obtention d'un médicament destiné à un traitement propre à la prévention et à la limitation de la libération dans l'organisme des médiateurs chimiques responsables du déclenchement des phénomènes inflammatoires des muqueuses en général et plus particulièrement des muqueuses des voies respiratoires, notamment nasales et bronchiques, des muqueuses buccales, vaginales, intestinales et anales.

5. Utilisation des solutions salines selon la revendication 1 pour l'obtention d'un médicament destiné à un traitement propre à prévenir et à limiter la libération, sous l'influence de substances d'origine bactérienne, de virus, bactéries, pollens et autres éléments irritants et allergisants transportés par l'air respiré, des médiateurs chimiques responsables du déclenchement des phénomènes inflammatoires des muqueuses des voies respiratoires humaines.

6. Utilisation des solutions salines selon la revendication 1 pour l'obtention d'un médicament destiné au traitement céruménolytique du conduit auditif externe, autrement dit à un traitement de dissolution des bouchons de cérumen.

7. Médicament à base d'une solution saline hypoosmotique selon la revendication 1 propre à prévenir et à limiter la libération dans l'organisme des médiateurs chimiques responsables du déclenchement des phénomènes inflammatoires des muqueuses en général et plus particulièrement des muqueuses des voies respiratoires, notamment nasales et bronchiques, des muqueuses buccales, vaginales, intestinales et anales.

8. Médicament à base d'une solution saline hypoosmotique selon la revendication 1, propre à prévenir et à limiter la libération, sous l'influence de substances d'origine bactérienne, de virus, bactéries, pollens et autres éléments irritants et allergisants transportés par l'air respiré, des médiateurs chimiques responsables du déclenchement des phénomènes inflammatoires des muqueuses des voies respiratoires humaines.

9. Médicament céruménolytique à base d'une solution saline hypoosmotique selon la revendication 1.

10. Procédé de préparation des solutions salines hypoosmotiques selon la revendication 1, **caractérisé par le fait que**, successivement,
- on prélève, en tant que matière première, de l'eau de mer d'une teneur en sels supérieure à 32 g/l avantageusement par une profondeur de 5 à 10 mètres dans une zone à forts mouvements de courant,
- on analyse et décante cette eau,
- on désode l'eau décantée par la technique d'électrodialyse jusqu'à ce que son osmolarité soit amenée à une valeur comprise entre 100 et 305 mOs/kg,
- on filtre et
- on stocke le produit obtenu dans des conditions stériles en cuve en acier inoxydable.

## Patentansprüche

1. Salzlösungen, **dadurch gekennzeichnet, daß** sie hypoosmotisch sind und daß sie aufweisen:
- einen pH-Wert von 7,0 bis 8,30,
- einen spezifischen elektrischen Widerstand von 52 bis 370 Ω und vorzugsweise von 57 bis 85 Ω,
- eine Dichte von 1,002 bis 1,008,
- einen Trockenmassenanteil von 0,5% bis 1%,
- eine Osmolarität, die zwischen 100 mOsmol/kg und 305 mOsmol/kg (Milliosmol/kg), vorzugsweise zwischen 140 und 240 mOsmol/kg, liegt, und
- eine chemische Zusammensetzung, deren hauptsächliche Elemente sich aus der nachfolgenden Tabelle I ergeben:
**TABELLE I**
| | |
|---|---|
| Natrium (Na) | von 600 bis 2000 mg/l |
| Kalium (K) | von 6 bis 40 mg/l |
| Chloride (Cl) | von 2000 bis 5800 mg/l |
| Calzium (Ca) | von 200 bis 300 mg/l |
| Magnesium (Mg) | von 1000 bis 1200 mg/l |
| Sulfate (SO4) | von 2000 bis 3000 mg/l |

2. Medikament, **dadurch gekennzeichnet, daß** es auf Basis einer hypoosmotischen Salzlösung ist und daß es aufweist:
- einen pH-Wert von 7,0 bis 8,30,
- einen spezifischen elektrischen Widerstand von 52 bis 370 Ω und vorzugsweise von 57 bis 85 Ω,
- eine Dichte von 1,002 bis 1,008,
- einen Trockenmassenanteil von 0,5% bis 1%,
- eine Osmolarität, die zwischen 100 mOsmol/kg und 305 mOsmol/kg (Milliosmol/kg), vorzugsweise zwischen 140 und 240 mOsmol/kg, liegt, und
- eine chemische Zusammensetzung, deren hauptsächliche Elemente sich aus der nachfolgenden Tabelle I ergeben:
**TABELLE I**
| | |
|---|---|
| Natrium (Na) | von 600 bis 2000 mg/l |
| Kalium (K) | von 6 bis 40 mg/l |
| Chloride (Cl) | von 2000 bis 5800 mg/l |
| Calzium (Ca) | von 200 bis 300 mg/l |
| Magnesium (Mg) | von 1000 bis 1200 mg/l |
| Sulfate (SO4) | von 2000 bis 3000 mg/l |

3. Verwendung der Salzlösungen nach Anspruch 1 als Medikament.

4. Verwendung der hypoosmotischen Salzlösungen nach Anspruch 1 zur Gewinnung eines Medikaments, das für eine Behandlung bestimmt ist, die zur Verhinderung und zur Begrenzung der Freisetzung der chemischen Mediatoren im Organismus geeignet ist, die für die Auslösung der Entzündungserscheinungen der Schleimhäute im allgemeinen und vor allem der Schleimhäute der Atemwege, insbesondere der Nase und der Bronchien, der Mund-, Vaginal- Darm- und Afterschleimhäute verantwortlich sind.

5. Verwendung der Salzlösungen nach Anspruch 1 zur Gewinnung eines Medikaments, das für eine Behandlung bestimmt ist, die zum Verhüten und zum Begrenzen der unter dem Einfluß von Substanzen bakteriellen Ursprungs, von Viren, Bakterien, Pollen oder anderen irritierenden und allergisierenden Elementen, die durch die eingeatmete Luft transportiert werden, ablaufenden Freisetzung der chemischen Mediatoren geeignet ist, die für die Auslösung der Entzündungserscheinungen der Schleimhäute der menschlichen Atemwege verantwortlich sind.

6. Verwendung der Salzlösungen nach Anspruch 1 zur Gewinnung eines Medikaments, das für die Ohrenschmalzbehandlung des äußeren Gehörgangs bestimmt ist, mit anderen Worten, für eine Behandlung zur Auflösung von Ohrenschmalzpfropfen.

7. Medikament auf Basis einer hypoosmotischen Salzlösung nach Anspruch 1, das zum Verhüten und zum Begrenzen der Freisetzung der chemischen Mediatoren im Organismus geeignet ist, die für die Auslösung der Entzündungserscheinungen der Schleimhäute im allgemeinen und vor allem der Schleimhäute der Atemwege, insbesondere der Nase und Bronchien, der Mund-, Vaginal-, Darm- und Afterschleimhäute verantwortlich sind.

8. Medikament auf Basis einer hypoosmotischen Salzlösung nach Anspruch 1, das zum Verhüten und zum Begrenzen der unter dem Einfluß von Substanzen bakteriellen Ursprungs, von Viren, Bakterien, Pollen oder anderen irritierenden und allergisierenden Elementen, die durch die eingeatmete Luft transportiert werden, ablaufenden Freisetzung der chemischen Mediatoren geeignet ist, die für die Auslösung der Entzündungserscheinungen der Schleimhäute der menschlichen Atemwege verantwortlich sind.

9. Ohrenschmalz-Medikament auf Basis einer hypoosmotischen Salzlösung nach Anspruch 1.

10. Verfahren zur Aufbereitung der hypoosmotischen Salzlösungen nach Anspruch 1, **dadurch gekennzeichnet, daß** nacheinander
- als erster Stoff Meerwasser mit einem Gehalt an Salzen von mehr als 32 g/l vorteilhaft aus einer Wassertiefe von 5 bis 10 Meter in einer Zone mit starken Strömungsbewegungen entnommen wird,
- dieses Wasser analysiert und geklärt wird,
- dem geklärten Wasser durch die Elektrodialysetechnik Natrium entzogen wird, bis seine Osmolarität auf einen Wert gebracht ist, der zwischen 100 und 305 mOsmol/kg liegt,
- gefiltert wird, und
- das gewonnene Produkt unter sterilen Bedingungen in einem Tank aus nichtoxidierbarem Stahl eingelagert wird.

## Claims

1. A saline solution, **characterized in that** it is hypoosmotic and **in that** it has:
- a pH of 7.0 to 8.30,
- a resistivity of 52 to 370 Ω and preferably of 57 to 85 Ω,
- a density of 1.002 to 1.008,
- a dry matter content of 0.5% to 1%,
- an osmolarity of between 100 mOs and 305 mOs/kg (milliosmol/kg), preferably between 140 and 240 mOs/kg, and
- a chemical constitution of which the principal elements result from Table I below:
**TABLE I**
| | |
|---|---|
| Sodium (Na) | from 600 to 2 000 mg/l |
| Potassium (K) | from 6 to 40 mg/l |
| Chlorides (Cl) | from 2 000 to 5 800 mg/l |
| Calcium (Ca) | from 200 to 300 mg/l |
| Magnesium (Mg) | from 1 000 to 1 200 mg/l |
| Sulfates (SO₄) | from 2 000 to 3 000 mg/l |

2. A medicine, **characterized in that** it is based on a hypoosmotic saline solution and **in that** it has:
- a pH of 7.0 to 8.30,
- a resistivity of 52 to 370 Ω and preferably of 57 to 85 Ω,
- a density of 1.002 to 1.008,
- a dry matter content of 0.5% to 1%,
- an osmolarity of between 100 mOs and 305 mOs/kg (milliosmol/kg), preferably between 140 and 240 mOs/kg, and
- a chemical constitution of which the principal elements result from Table I below:
**TABLE I**
| | |
|---|---|
| Sodium (Na) | from 600 to 2 000 mg/l |
| Potassium (K) | from 6 to 40 mg/l |
| Chlorides (Cl) | from 2 000 to 5 800 mg/l |
| Calcium (Ca) | from 200 to 300 mg/l |
| Magnesium (Mg) | from 1 000 to 1 200 mg/l |
| Sulfates (SO₄) | from 2 000 to 3 000 mg/l |

3. The use as a medicine of the saline solutions as claimed in claim 1.

4. The use of the hypoosmotic saline solutions as claimed in claim 1 for the production of a medicine intended for a treatment suitable for the prevention and for the limitation of the release, into the body, of the chemical mediators responsible for triggering the phenomena causing inflammation of the mucous membranes in general and more particularly of the mucous membranes of the respiratory, in particular nasal and bronchial, tracts, of the buccal, vaginal, intestinal and anal mucous membranes.

5. The use of the saline solutions as claimed in claim 1 for the production of a medicine intended for a treatment suitable for preventing and limiting the release, under the influence of substances of bacterial origin, viruses, bacteria, pollen and other irritant and allergenic constituents transported by the air inhaled, of the chemical mediators responsible for triggering the phenomena causing inflammation of the mucous membranes of the human respiratory tracts.

6. The use of the saline solutions as claimed in claim 1 for the production of a medicine intended for the cerumenolytic treatment of the external auditory canal, in other words for a treatment for dissolving cerumen plugs.

7. A medicine based on a hypoosmotic saline solution as claimed in claim 1 suitable for preventing and limitating the release, into the body, of the chemical mediators responsible for triggering the phenomena causing inflammation of the mucous membranes in general and more particularly of the mucous membranes of the respiratory, in particular nasal and bronchial, tracts, of the buccal, vaginal, intestinal and anal mucous membranes.

8. A medicine based on a hypoosmotic saline solution as claimed in claim 1, suitable for preventing and limiting the release, under the influence of substances of bacterial origin, viruses, bacteria, pollen and other irritant and allergenic constituents transported by the air inhaled, of the chemical mediators responsible for triggering the phenomena causing inflammation of the mucous membranes of the human respiratory tracts.

9. A cerumenolytic medicine based on a hypoosmotic saline solution as claimed in claim 1.

10. A method of preparing the hypoosmotic saline solutions as claimed in claim 1, **characterized in that**, successively,
- there is collected, as raw material, sea water having a salts content greater than 32 g/l, advantageously at a depth of 5 to 10 meters in a zone with strong movements of current,
- this water is analyzed and allowed to settle,
- sodium-salt is removed from the water, which has been allowed to settle, by the electodialysis technique until its osmolarity is brought to a value of between 100 and 305 mOs/kg,
- the medium is filtered, and
- the product obtained is stored under sterile conditions in a stainless steel tank.
